## Europäisches Patentamt

## European Patent Office

## Office européen des brevets

(19)

(11) Publication number: **0 195 859**
A2

# (12) EUROPEAN PATENT APPLICATION

(21) Application number: **85116648.8**

(22) Date of filing: **30.12.85**

(51) Int. Cl.⁴: **C 09 J 3/14,** C 09 J 7/00
// A61N1/04

(30) Priority: **27.03.85 US 716590**

(43) Date of publication of application: **01.10.86**
**Bulletin 86/40**

(84) Designated Contracting States: **AT CH DE FR GB IT LI SE**

(71) Applicant: **Norwood Industries, Inc., 100 North Morehall
Road, Malvern Pennsylvania 19355 (US)**

(72) Inventor: **Bowditch, William Raymond, 1412 Carter
Place, West Chester Pennsylvania 19380 (US)**
Inventor: **Rybalka, Borys, 9739 Redd Rambler Place,
Philadelphia Pennsylvania 19115 (US)**

(74) Representative: **Wuesthoff, Franz, Dr.-Ing. et al,
Patentanwälte Wuesthoff -v. Pechmann-Behrens-Goetz
Schweigerstrasse 2, D-8000 München 90 (DE)**

(54) **Conductive adhesives.**

(57) The present invention is a conductive adhesive composition containing pressure sensitive adhesive solids and a plurality of silver-coated spheres. The spheres are distributed throughout the composition in a packing configuration which yields a conductive network of adjacent silver surfaces. The composition demonstrates both good cohesive strength–comparable to that of the constituent pressure sensitive adhesive solids taken alone–and good conductivity. Good cohesive strength and conductivity are accomplished by controlling the amount, the average diameter and the particle size distribution of the silver-coated glass spheres in relation to the amount of adhesive solids present in the composition. The composition has utility in a wide variety of industrial and medical applications and is particularly well suited for use in diagnostic procedures such as electro-encephalography and cardiography. A laminated electrode facing incorporating the conductive adhesive is also disclosed.

PATENTANWÄLTE

# WUESTHOFF - v. PECHMANN - BEHRENS - GOETZ

### EUROPEAN PATENT ATTORNEYS

DR.-ING. FRANZ WUESTHOFF (1927-1956)
DR. PHIL. FREDA WUESTHOFF (1927-1956)
DIPL.-ING. GERHARD PULS (1952-1971)
DIPL.-CHEM. DR. E. FREIHERR VON PECHMANN
DR.-ING. DIETER BEHRENS
DIPL.-ING. DIPL.-WIRTSCH.-ING. RUPERT GOETZ

D-8000 MÜNCHEN 90
SCHWEIGERSTRASSE 2

TELEFON: (089) 66 20 51
TELEGRAMM: PROTECTPATENT
TELEX: 5 24 070
TELEFAX: VIA (089) 2 71 60 63 (III)

NEUE TELEFAX-NR.:
(089) 66 39 36 (III)

Norwood Ind.
Our File: EP-59 754

## CONDUCTIVE ADHESIVES

## Field of the Invention

The present invention relates to conductive adhesives, and more particularly, to adhesives having conductivity attributable to a network of silver-coated glass spheres therein.

## Introduction

Conductive adhesives are required in a broad spectrum of industrial applications. A few of the many industries which demand conductive adhesives are the electronics, telecommunications, equipment and product manufacturing and construction industries, along with the health care industry and its diverse requirements for conductive adhesives in medical, surgical and diagnostic procedures. In any given application, a conductive adhesive must demonstrate both the conductivity and the cohesive strength required by that application; a product which does not satisfy both the conductivity and the cohesion requirements is unsuitable for use.

Products which are both adhesive and conductive are, unfortunately, difficult to formulate. Formulation is difficult because the addition of a conductive element to an adhesive invariably lowers the performance of the adhesive, and the presence of an adhesive adjacent to a conductive material predictably lowers its conductivity. As a result, although certain conductive adhesives are known in the art, those which are highly conductive generally are low performance bonding agents, and superior bonding agents consistently demonstrate inferior—and sometimes only threshold—conductivity.

## Description of the Prior Art

Traditionally, electrolyte paste compositions have been used, in the health care industry, as conductive media and short-term bonding agents. Although use of these pastes has been widespread, particularly in securing diagnostic electrodes to human skin, numerous disadvantages inhere in these electrolyte compositions. As bonding agents, the pastes provide extremely low cohesive strength. As conductive media, the pastes seldom demonstrate good conductivity, despite the constituent electrolyte. In addition, the pastes leave a thick, irritating residue on the skin and, even more significantly, cannot be prepared until shortly before use. No advance preparation is possible because, unless the compositions are hermetically sealed and stored in a vacuum, the conductivity of the pastes is largely destroyed by oxidation and/or dehydration. Electrolyte pastes have proved themselves, as a result, as inferior conductive adhesives suitable only for limited uses.

Non-electrolyte conductive compositions are known in which a plurality of conductive particles is distributed within the composition to yield a conductive network throughout. For example, U.S. -A- 4,231,901 discloses an electrically conductive foam having electrically conductive particulate material distributed therein. U.S. -A- 3,725,308 likewise discloses an electrically conductive mass including finely divided particles of nickel or cobalt coated with gold or silver and held together by an organic or inorganic binder or matrix. In addition, conductive compositions are known wherein silver-coated ceramic spheres are incorporated into soft conductive materials, as disclosed in U.S. -A- 4,098,945 .

Silver-coated glass spheres are disclosed as useful conductive filler for adhesive compositions in the brochure "Silver Coated Glass Spheres, A Low Cost Alternative to Pure Silver in Conductive and Shielding Applications," distributed by Potters Industries, Inc. No prior art adhesive containing conductive particles has, however, demonstrated simultaneous conductivity good cohesive strength: when conductive particles have been admixed into an adhesive in an amount sufficient to yield a conductive network therein, the cohesive strength of the adhesive has been largely destroyed by the particulate filler. Likewise, when the conductive particles have been limited to amounts which preserve the cohesive strength of the adhesive, the composition has demonstrated inferior or threshold conductivity due to insufficient conductive filler.

A need remains, therefore, for a pressure sensitive adhesive composition having conductive filler therein which demonstrates both good conductivity and cohesive strength comparable to the cohesive strength demonstrated by its constituent pressure sensitive adhesive solids.

## Summary of the Invention

In order to meet this need, the present invention provides a conductive adhesive composition in which controlled amounts of specified silver-coated spheres are admixed with pressure sensitive adhesive solids. The composition demonstrates cohesive strength comparable to that of its constituent pressure sensitive adhesive solids, and good conductivity as well. Good conductivity and cohesive strength are achieved by controlling the amount, the average diameter and the particle size distribution of the silver-coated spheres in relation to the proportionate amount of adhesive solids present in the composition.

Preferably, the silver-coated spheres are silver-coated glass spheres having about 12 percent by weight silver and about 88 percent by weight glass. Specifically, in the preferred embodiment of the invention, the composition includes between 65 and 90 percent by weight adhesive solids and between 10 and 35 percent by weight silver-coated glass spheres. The spheres have an average diameter of 15 μm and a particle size distribution between 1 and 30 μm.

In the alternate embodiment of the invention, the composition includes between 25 and 35 percent by weight pressure sensitive adhesive solids and between 65 and 75 percent by weight silver-coated glass spheres. The spheres, in the alternate embodiment of the invention, have an average diameter of 25 μm and a particle size distribution between 5 and 45 μm. The composition has utility in a wide variety of industrial and medical applications, and is particularly well suited for use in diagnostic procedures such as electro- encephalography and cardiography.

## Detailed Description of the Invention

The present conductive adhesive formulation may be admixed in accordance with techniques known in the adhesive arts. Generally, the correct amounts of the components of the composition are admixed in a suitable laboratory or production vessel. The desired amount of a solvent inert to the reaction is added to control viscosity. After thorough mixing, the conductive adhesive may be applied to a wide variety of substrates and materials and cured to a pressure sensitive adhesive layer or film.

The conductive adhesive composition includes a pressure sensitive adhesive component. Any pressure sensitive adhesive is suitable for use in the present invention as long as the cohesive strength of the adhesive solids per se is adequate for a given application and as long as the adhesive solids are present in the composition in the required proportions relative to the silver-coated spheres. Typical pressure sensitive adhesives include the acrylic polymeric adhesive compositions such as the acrylic and modified acrylic polymer and multipolymer adhesives (GELVA® RA-737 Monsanto and DURO-TAK® National Starch & Chemical Corporation). Preferably, the pressure sensitive adhesive component is a self-curing acrylic polymeric adhesive composition. Included among the suitable acrylic polymer adhesives are the 1) polymers resulting from the reaction of an acrylic interpolymer containing hydroxyl or carboxyl groups with a polymetaloxane, ( U.S. -A- 4,185,051 and 4,234,660), 2) polymers resulting from the admixture of acid-epoxy monomer resin, containing esters of acrylic or methacrylic acid, with 1,3 bis(dimethylamino)-2-hydroxypropane, ( U.S. -A- 3,-893,982 and 3,903,057 ) and 3) polymers resulting from the admixture of N,N-diacetonylacrylamide with esters of acrylic acid or methacrylic acid, ( U.S. -A- 3,900,610 ).

Additional suitable acrylic polymer adhesives include the 1) polymers resulting from the admixture of a linear copolymer of maleic anhydride, vinyl acetate, octyl acrylate and ethyl acrylate, with a crosslinkable acrylic copolymer ( U.S. -A- 3,257,478 ) ; 2) polymers resulting from the admixture of copolymers of vinyl acetate and acrylic or methacrylic alkyl esters with a crosslinkable acrylic copolymer ( U.S. -A- 3,222,419) ; 3) allyl acrylate copolymers with pendant allyl groups, ( U.S. -A- 4,234,662 4) acrylic polymers admixed with chlorosulfonated polyethylene initiators and a polymerizable vinyl compound (U.S.-A- 4,113,792 and 4,170,612 ); 5) polymers of alkyl acrylate or methacrylate with quinone type ultraviolet sensitizers, ( U.S. -A- 4,069,123 ); 6) interpolymers of active hydrogen acrylic monomers and metal alkoxides, (U.S.-A- 3,769,254 ) ; 7) cyanoacrylate polymers (U.S.-A- 3,701,758 ) and 8) interpolymers of alkyl acrylates, at least one vinyl ester of a saturated monocarboxylic acid, and a small amount of $\alpha / \beta$ -ethylenically unsaturated dicarboxylic acid or anhydride, (U.S.-A- 3,697,618 ).

Water-based pressure sensitive adhesive compositions are also well-suited to the practice of the invention. Preferably, these water-based systems are self-curing. An example of such a water-based pressure sensitive adhesive system is disclosed in U.S.-A- 4,179,415 which discloses a latex component in admixture with a high solids tackifier resin such that the resin is dispersed in the aqueous system of the latex.

The silver-coated spheres are solid spheres onto which has been vacuum deposited a controlled amount of metallic silver. Any given sample of a quantity of these spheres has an average particle diameter and a particle size distribution. As used herein, the particle size distribution is the range of particle diameters which embraces approximately 80% of the particle diameters in the sample. The average diameter is both the actual average diameter of the plurality of spheres in the sample and the mid-point of the range defined by the particle size distribution. For the purposes of the invention, therefore, the quantity of spheres added to the pressure sensitive adhesive solids has both a specified average diameter and a specified particle size distribution.

The spheres themselves may be fabricated of any chemically inert ceramic, metal or other solid. Preferably, however, the spheres are made of chemically and biologically inert glass. Suitable glass formulations are those containing potash-lime glass, soda-lime glass, potash-lead glass and the various "bottle glasses," or silicates of sodium, potassium, calcium and aluminum. After the glass is formed into rounds, sufficient silver is vacuum deposited onto the spheres to yield a product having between about 85 and about 90 percent by weight glass and between about 10 and about 15 percent by weight silver. Preferably, the silver-coated glass spheres include about 12 percent by weight silver and about 88 percent by weight glass. Silver-coated glass spheres may be fabricated by methods known in the art or may be obtained from manufacturers such as Potters Industries, Inc.

A wide variety of solvents may be introduced into the admixture of pressure sensitive adhesive solids and the silver-coated glass spheres. Solvents are added in an amount suitable to provide a workable viscosity or in a controlled amount to yield an admixture having a specified viscosity for a particular application. Preferably, sufficient solvent is added to yield an admixture having a viscosity between 1000 and 6000 mPa·s

$<$ 1000 mPa·s the spheres tend to settle out of suspension before the adhesive can be cured. Solvents for pressure sensitive adhesive solids are known in the art and include the lower alkyl alcohols, such as ethanol, isopropanol and butanol, the lower alkyl acetates such as ethyl acetate, and the low-boiling solvents such as hexane, heptane and toluene. Frequently, however, commercially available pressure sensitive adhesives, such as those enumerated above, are sold in admixture with a standard amount of a suitable solvent inert to the reaction and, ordinarily, the practice of the present invention requires no solvent addition at all.

In the preferred embodiment of the invention, a mixture of adhesive solids and glass spheres is prepared wherein between 65 and 90 percent by weight of the composition is adhesive solids and between 10 and 35 percent by weight of the composition is silver-coated glass spheres, net after solvent removal. (Because solvents may be admixed into the composition in any desired amount, and because the solvents are removed when the adhesive is cured, the formulation of the present invention is specified by weight percent of its solids content, net after solvent removal.) The silver-coated glass spheres have an average diameter of 15 μm and a particle size distribution between 1 and 30 μm. The resultant admixture of adhesive solids and silver-coated glass spheres yields a conductive adhesive having both good conductivity and good cohesive strength. Preferably, the composition contains between 67 and 87 percent by weight adhesive solids and between 13 and 33 percent by weight silver-coated glass spheres.

In the alternate embodiment of the invention, a mixture of adhesive solids and glass spheres is prepared wherein between 25 and 35 percent by weight pressure sensitive solids are admixed with between 65 and 75 percent by weight silver-coated glass spheres. These glass spheres have an average diameter of 25 $\mu$m and a particle size distribution between 5 and 45 $\mu$m. Preferably, the composition contains about 30 percent by weight adhesive solids and about 70 percent by weight silver-coated glass spheres. The resultant admixture of adhesive solids and glass spheres yields a conductive adhesive having both good conductivity and good cohesive strength. Although cohesive strength will be less, for any given pressure sensitive adhesive, than for the same adhesive formulated in accordance with the preferred embodiment of the invention, the composition is still far superior to prior art conductive adhesives. Specifically, the alternate composition is lower in cost than the preferred composition due to the decreased total amount of silver required on the fewer, larger spheres and is still superior in cohesive strength to prior art conductive adhesives in which the conductive particles do not satisfy the amount, average diameter and particle size distribution requirements of the preferred or the alternate embodiments of the present invention.

The amount, the average size and the particle size distribution of the silver-coated glass spheres are each critical to the present invention. In theory, although applicant does not intend to be bound by this theory, the simultaneous cohesion and conductivity demonstrated by the present formulation result from the packing configuration which results when the amount, average size and particle size distribution are each carefully controlled. The number of spheres present is limited enough to preserve the cohesive strength of the adhesive solids, yet still provides a continuous network of adjacent silver surfaces for reliable conductivity. In addition, the average diameter of the spheres (50 $\mu$m or less) is small enough relative to even a thin adhesive film that the tiny spheres do not interfere significantly with film formation, cohesion or conformability of the adhesive solids.

The average diameter and the quantity of glass spheres do not alone account for the superior characteristics of the present formulations, however, because the particle size distribution is critical to the packing configuration of the spheres within the composition. In theory, particles of uniform size do not pack sufficiently to form a good conductive network: except for the six contact points on each uniform sphere, the nonadjacent silver surfaces are separated by multiple non-conductive "void" spaces filled with adhesive solids. Particles of varied sizes, however, arrange themselves into a more dense packing configuration in which the smaller particles fill the voids remaining between the larger particles. Although the use of wide particle size distributions has been successful in nonadhesive conductive materials, the resultant dense packing configurations tend to drastically reduce the cohesive strength of adhesive compositions. Applicant has, by contrast, identified the specific particle size distribution which, in combination with particles of a specified diameter and amount, yields a conductive adhesive composition which demonstrates both good conductivity and good cohesive strength.

The present conductive adhesive is particularly well-suited for use in laminated electrode facings for electrophysiologic electrode plates. These laminated facings may be prepared well in advance of use and may be stored in either ordinary inventory (for use with conventional electrodes) or in position on the surface of a disposable, ready-to-use electrode plate. The laminated facings include a cured film of one of the present conductive adhesive formulations and a release liner covering the outer surface of the film. Optionally, the inner surface of the conductive adhesive film may be laminated to a conductive sheet such as a continuous or porous metallic or metallized sheet material. The electrode plate and its projecting leads may thus adhere directly to the inner surface of the conductive adhesive film, or the electrode plate may rest adjacent the metallic or metallized sheet with the projecting leads piercing through it to the extent necessary to establish good electrical contact.

More particularly, the laminated electrode facings may be made and used as follows. A release liner is fashioned of a strong paper, preferably calendered of bleached draft fibers, by coating at least one side of the paper with a polyethylene coating and then a finishing coat of silicone. The treated surface of the release liner is then coated with a conductive adhesive composition of the present invention by knife-coating or related techniques,

and cured to a film having a thickness between / 0,0127 and 254 μm (0.005 and 10 mils). The facings may then be cut and packaged by various methods known in the pressure sensitive adhesive film art, or may be applied directly to permanent or disposable electrode plates.

The optional conductive sheet may be added as follows. A metallic or metallized sheet is prepared having a conductive surface on at least one side. Preferably, the sheet is an aluminized veil, which is a gauze or other porous substrate onto which has been vacuum deposited a biologically inert aluminum coating. Aluminized veils are known in the art and are available (from Lohmann GmbH & Co., KG ). The veil may be aluminized on one or both sides. Other suitable metallic or metallized sheet materials include the metallic foil and foil laminates; any conductive sheet material is suitable for use as long as at least one side of the sheet is conductive and as long as the structure of the sheet permits the electrode leads to contact a conductive surface of the laminated electrode facing, i.e., by attaching to or piercing through the sheet material to establish electrical contact with the conductive sheet or adhesive.

The conductive sheet is then laminated with the conductive adhesive film by compressing the inner surface of the adhesive film with the conductive surface of the conductive sheet. The resulting laminate is then cut and packaged by methods known in the art. The laminated electrode facing thus includes 3 . layers: a conductive sheet, a conductive adhesive film, and a release liner. The electrode facings may be stored for later application to permanent or disposable electrode plates, or may be inventoried for use as attachment pads for direct connection to electrode leads without need for conventional electrode plates at all.

Preferably, the conductive sheet is an aluminized veil having vacuum deposited aluminum on only one side. The incorporation of this particular aluminized veil affords three advantages to the laminated electrode facing. First, the electric leads of the electrode may easily pass through the interstices of the aluminized veil to contact the aluminized surface, the conductive adhesive, or both. Second, small amounts of the pressure sensitive adhesive solids may deform to extend through the interstices of the veil to provide cohesion between the conductive sheet and the electrode plate if an electrode plate is present. (These two advantages are, of course, common to all aluminized veils.)  Finally, because the entire aluminized surface is covered with adhesive solids, the aluminum does not oxidize even after extended storage and handling of the laminated electrode facings.  The strictly impervious packaging required for electrode facings having exposed aluminum surfaces is, therefore, unnecessary.  The resultant product has a shelf-life of at least  2  years regardless of the packaging used or the nature of ambient (i.e., 15      to 30° C ) storage conditions.

Ideally, the electrode facing will have a total thickness of less than ( 10      254 μm mils ) . The release liner is preferably (4.0 to 5.0 mils) 0.102-0.127 mm thick, the conductive adhesive film is preferably (2 mils) 51 μm thick and the conductive sheet—whether aluminized veil or foil—is preferably less than (1 mil) 25,4 μm thick.  Aluminum, furthermore, is the preferred metal for use in the conductive sheet material because it demonstrates low toxicity without the prohibitive costs associated with comparable amounts of silver or gold.

The invention is further illustrated by reference to the following examples.

## EXAMPLE I

100 g  of silver-coated glass spheres were weighed in a tared receptacle and transferred to a mixing vessel.  The silver-coated glass spheres had a density of 2.7 g/cm$^3$,                12% of which was metallic silver and 88% of which was glass.  The spheres had an average diameter of 25  μm and a particle size distribution between         5  and 45 μm.      To the mixing vessel was added 80 g  of a modified acrylic pressure sensitive self-curing adhesive composition containing 50% adhesive solids, 22% isopropanol, 18% heptane, 7% ethyl acetate and 3% toluene.  10 g  of toluene were added to the vessel and the composition was mixed thoroughly.  The admixture was coated onto a release liner with a knife edge placed (0.009 mils) 0.23 μm above the release liner.  The coated film was dried at (160° F) 71 °C for 10 minutes and at 107°C (225° F)  for  6  minutes.   The dried film was completely cured and demonstrated both good conductivity and superior adhesion to aluminum foil.

## EXAMPLE II

15 g of silver-coated glass spheres were weighed in tared receptacle and transferred to a mixing vessel. The silver-coated glass spheres had a density of 2.7 g /cm$^3$, 12% of which was metallic silver and 88% of which was glass. The spheres had an average diameter of 15 μm and a particle size distribution between 1 and 30 μm. To the mixing vessel was added 60 g of the adhesive composition described in Example I, and the composition was mixed thoroughly. The resultant composition had a solids content of 2/3 adhesive solids and 1/3 silver-coated glass spheres by weight. The admixture was coated and cured, after which testing revealed a conductive, cohesive film.

## EXAMPLE III

The conductive adhesive composition was admixed in accordance with Example II, except that 5 g of the silver-coated glass spheres were substituted for the 15 g. The resultant film demonstrated good conductivity despite the decrease in silver-coated glass spheres, and exhibited good cohesive strength.

## EXAMPLE IV

10 g of the silver-coated glass spheres described in Example II were weighed and transferred to a mixing vessel. 96.8 g of a multipolymer acrylic resin composition were added to the vessel. The resin composition contained 30% by weight adhesive solids and the remaining 69% by weight of mixed solvents included 50 parts by weight ethyl acetate, 35 parts by weight ethanol and 15 parts by weight toluene. The resin composition and the glass spheres were admixed, yielding an adhesive composition having a viscosity of 1600 mPa·s. The admixture was coated onto a release liner with a knife edge placed 0.41 μm (0.016 mils) above the release liner. The resultant film was dried for 10 minutes at 71°C (160° F) and for 6 minutes at 105°C (220° F). The cured conductive adhesive composition demonstrated both good conductivity and good cohesive strength.

## EXAMPLE V

The admixture of Example I was coated onto a release liner to yield a film 0,254 mm (10 mils) thick. Samples of the film were cut, without removing the film from the release liner, into 25,4 x 25,4 mm segments. One segment was placed between 2 25,4 x 25,4 mm aluminum plates to which were attached the leads of an Ohmmeter. Measured resistivity was 0,062 $\Omega$/mm$^2$ (0,004 Ohms/in.$^2$) indicating high conductivity due to very low resistivity.

A different segment was tested in series with a mass of known resistivity. Resistivity of 9, 7,44, 3,56, 3,56 and 3,56 $\Omega$/cm$^2$ (58, 48, 23, 23 and 23 Ohms/in.$^2$) was observed at 0.1, 0.5, 1.0, 1.5 and 2.0 MHz, respectively.

A third segment was tested with the high frequency impedance meter. Test results demonstrated resistivity of 8,5, 7,6, 6,4 and 3,6 $\Omega$/cm$^2$ (55, 49, 41, 36 and 32 Ohms/in.$^2$) at 0.1, 0.5, 1.0, 1.5 and 2.0 MHz, respectively.

For comparison 2 salt electrolyte paste compositions commonly used in electro- encephalography and cardiography were tested. A 0,51 - 0,76 mm (20-30 mil) thickness of one electrolyte paste demonstrated resistivity of 13,2, 11,0, 5,4, 5,6 and 5,4 $\Omega$/cm$^2$ (85, 71, 35, 36 and 35 Ohms/in.$^2$) at 0.1, 0.5, 1.0, 1.5 and 2.0 MHz, respectively. A 0,51 - 0,76 mm (20-30 mil) thickness of the second electrolyte paste demonstrated resistivity of 16,9, 15,7, 11,9, 11,6 and 11,5 $\Omega$/cm$^2$ (109, 101, 77, 75 and 74 Ohms/in.$^2$) at 0.1, 0.5, 1.0, 1.5 and 2.0 MHz, respectively. The first electrolyte paste demonstrated resistivity of 8,9 $\Omega$/cm$^2$ (57.5 Ohms/in.$^2$) when tested with an Ohm meter, and the second electrolyte paste demonstrated resistivity of 11,1 $\Omega$/cm$^2$ (71.5 Ohms/in.$^2$), thus demonstrating commensurately higher conductivity than conductive electrolyte pastes known in the art.

## EXAMPLE VI

Conductive adhesive formulations were prepared in accordance with Examples II, III and IV, except that the adhesive composition of Example I was substituted for the resin composition of Example IV. Films 2 mils thick were then prepared from each of the formulations of Examples II and III and the modified formulation of Example IV. 25,4x25,4 mm samples were prepared from each of the films. Each sample was tested with an Ohm meter and the high frequency impedance meter.

The film sample, in accordance with Example II, demonstrated low resistivity $(0.0775 \Omega/mm^2)$ 0.005 Ohms/in.$^2$ when tested with an Ohm meter. The sample demonstrated resistivity of $(62, 4.8, 2.5, 2.5 \text{ and } 2.2 \Omega/cm^2)$ (40, 31, 16, 16 and 14 Ohms/in.$^2$) when tested with the high frequency impedance meter at 0.1, 0.5, 1.0, 1.5 and 2.0 MHz, respectively.

The film sample, in accordance with Example III, also demonstrated low resistivity $(0.0115 \Omega/mm^2)$ 0.001 Ohms/in.$^2$ when tested with an Ohm meter. The sample demonstrated resistivity of $(7.9, 6.0, 8.9, 3.4 \text{ and } 3.6 \Omega/cm^2)$ (51, 39, 25, 22 and 23 Ohms/in.$^2$) when tested with the high frequency impedance meter at 0.1, 0.5, 1.0, 1.5 and 2.0 MHz, respectively.

The film sample, in accordance with Example VI, demonstrated 0.004 $(0.062 \Omega/mm^2)$ Ohms/in.$^2$ resistivity (Ohm meter) and resistivity of $(6.4, 4.8, 2.6, 2.6 \text{ and } 2.5 \Omega/cm^2)$ (41, 31, 17, 17 and 16 Ohms/in.$^2$) when tested with the high frequency impedance meter at 0.1, 0.5, 1.0, 1.5 and 2.0 MHz, respectively.

The test results set forth in Examples I-VI are summarized in the following Table:

## Table I

### Resistivity

| | A | B | C | D | E | F | G | |
|---|---|---|---|---|---|---|---|---|
| Ohm Meter | 0.004 | 0.004 | 0.005 | 0.001 | 0.004 | 57.5 | 71.5 | $\Omega/in^2$ |
| | 0.00062 | 0.00162 | 0.000775 | 0.000155 | 0.00062 | 8.9 | 11.1 | $\Omega/cm^2$ |
| Frequency in MHz | | | | | | | | |
| 0.1M | 58 | 55 | 40 | 51 | 41 | 85 | 109 | |
| 0.5M | 48 | 49 | 31 | 39 | 31 | 71 | 101 | |
| 1.0M | 23 | 41 | 16 | 25 | 17 | 35 | 77 | |
| 1.5M | 23 | 36 | 16 | 22 | 17 | 36 | 75 | |
| 2.0M | 23 | 32 | 14 | 23 | 16 | 32 | 74 | |

in which

A is the formulation of Example I;
B is the formulation of Example I;
C is the formulation of Example II;
D is the formulation of Example III;
E is the modified formulation of Example IV; and
F is the first and
G is the second electrolyte paste composition described in Example V

## EXAMPLE VII

Onto a sheet of release paper(5 mils)thick 0,127 mm was coated a 2 mil 51 µm thickness of the conductive adhesive formulation of Example III. The aluminized surface of an aluminized veil having an aluminum coating on only one side was placed adjacent the exposed surface of the conductive adhesive film. The aluminized veil readily adhered to the conductive adhesive film and a three-layered laminate resulted. The laminate was cut into individual electrode facings by stamping with a die coated with a silicone release agent. The electrode facings were tested and demonstrated good adhesion to skin and superior electrical conductivity. Storage of the facings at ambient temperature for 18 months did not result in any perceptible deterioration in either conductivity or cohesive strength.

PATENTANWÄLTE

WUESTHOFF - v. PECHMANN - BEHRENS - GOETZ

EUROPEAN PATENT ATTORNEYS

0195859

DR.-ING. FRANZ WUESTHOFF
DR. PHIL. FREDA WUESTHOFF (1927-1956)
DIPL.-ING. GERHARD PULS (1952-1971)
DIPL.-CHEM. DR. E. FREIHERR VON PECHMANN
DR.-ING. DIETER BEHRENS
DIPL.-ING. DIPL.-WIRTSCH.-ING. RUPERT GOETZ

D-8000 MÜNCHEN 90
SCHWEIGERSTRASSE 2

TELEFON: (089) 66 20 51
TELEGRAMM: PROTECTPATENT
TELEX: 5 24 070
TELEFAX: VIA (089) 2 71 60 63 (III)

Norwood Ind.
Our File: EP-59 754

Claims

1. A conductive adhesive composition comprising an admixture of pressure sensitive adhesive solids and a plurality of silver-coated glass spheres, wherein the admixture contains between 65 and percent by weight of said adhesive solids and between 10 and percent by weight of said spheres, the spheres having an average diameter of 15 μm and a particle size distribution between 1 and 30 μm.

2. The composition of claim 1 wherein the admixture contains between 67 and 87 percent by weight of the adhesive solids and between 13 and 33 percent by weight of the spheres.

3. The composition of claim 2 wherein the spheres consist essentially of about 88 percent by weight glass and about 12 percent by weight silver.

4. A conductive adhesive composition comprising and admixture of pressure sensitive adhesive solids and a plurality of silver-coated glass spheres, wherein the admixture contains between 25 and 35 percent by weight of the adhesive solids and between 65 and 75 percent by weight of the spheres, the spheres having an average diameter of 25 μm and a particle size distribution between 5 and 45 μm.

5. The composition of claim 4 wherein the admixture contains about 30 percent by weight of said adhesive solids and about 70 percent by weight of the spheres.

6. The composition of claim 5 wherein the spheres consist essentially of about 88 percent by weight glass and about 12 percent by weight silver.

7. The composition of claim 3 or 6 wherein the pressure sensitive adhesive solids consist essentially of acrylic polymeric adhesive solids.

8. A conductive adhesive laminate comprising a film formed from the composition of claim 1 and a release liner adjacent to one surface of said film.

9. The conductive adhesive laminate of claim 8 wherein the surface of the film opposite to the release liner is positioned adjacent to a conductive sheet material.

10. The conductive adhesive laminate of claim 9 wherein the conductive sheet material is an aluminized veil.

11. The conductive adhesive composition of claim 10 wherein the film is about 51 mm in thickness.

12. The conductive adhesive laminate of claim 11 wherein the aluminized veil has an aluminum coating on only one side, whereby the adhesive solids substantially completely cover said aluminum coating.